(19) Europäisches Patentamt

European Patent Office

Office européen des brevets

(11) **EP 1 101 486 B1**

(12) **FASCICULE DE BREVET EUROPEEN**

(45) Date de publication et mention
de la délivrance du brevet:
**26.07.2006 Bulletin 2006/30**

(51) Int Cl.:
*A61Q 5/06* (2006.01)   *A61K 8/72* (2006.01)

(21) Numéro de dépôt: **00403135.7**

(22) Date de dépôt: **10.11.2000**

(54) **Composition cosmétique comprenant un polymère aux caractéristiques particulières et un polymère épaississant**

Kosmetische Zusammensetzung, die ein Polymer mit speziellen Eigenschaften und ein verdickendes Polymer enthält

Cosmetic composition containing a polymer with specific characteristics and a thickener

(84) Etats contractants désignés:
**AT BE CH CY DE DK ES FI FR GB GR IE IT LI LU MC NL PT SE TR**

(30) Priorité: **19.11.1999 FR 9914589**

(43) Date de publication de la demande:
**23.05.2001 Bulletin 2001/21**

(73) Titulaire: **L'ORÉAL**
**75008 Paris (FR)**

(72) Inventeurs:
• **Rollat-Corvol, Isabelle**
**75017 Paris (FR)**
• **Cothias, Pascale**
**78180 Montigny-le-Bretonneux (FR)**

(74) Mandataire: **Bourdeau, Françoise**
**L'OREAL - D.I.P.I.**
**25-29 Quai Aulagnier**
**92600 Asnières (FR)**

(56) Documents cités:
**EP-A- 0 628 304       FR-A- 2 108 635**
**FR-A- 2 730 162       FR-A- 2 750 601**
**FR-A- 2 786 391       FR-A- 2 786 392**
**GB-A- 1 530 517**

• **"Avalure Film Forming Polymers for Personal Care Applications" INTERNET WWW.CARBOPOL.COM/ TECHDATA/PDF/TDS248.PDF, 28 avril 1997 (1997-04-28), XP002109281**

## Description

L'invention a pour objet une composition cosmétique comprenant, dans un milieu cosmétiquement acceptable, au moins un polymère filmogène (A) aux caractéristiques particulières et au moins un polymère (B) épaississant réticulé comprenant au moins un monomère d'acide (méth-)acrylique. Elle vise également un procédé de mise en forme ou de maintien des cheveux à l'aide de cette composition ainsi que son utilisation pour la formulation de produits de coiffage, notamment de gels, en vue d'obtenir le maintien ou la mise en forme de la coiffure.

Les compositions conformes à l'invention peuvent être appliquées sur la peau, les ongles, les lèvres, et les fibres kératiniques, comme les cheveux, les sourcils et les cils.

Au sens de la présente invention, on entend par « composition de coiffage », toute composition destinée à fixer et/ou à maintenir la forme de la coiffure.

Les produits capillaires pour la fixation des cheveux les plus répandus sur le marché de la cosmétique sont constitués d'une solution le plus souvent alcoolique ou hydroalcoolique et d'un polymère filmogène soluble dans l'eau ou dans l'alcool, en mélange avec divers adjuvants cosmétiques.

Toutefois, ces formulations capillaires telles que les mousses, gels et surtout les sprays et les laques aérosols destinées à maintenir la forme de la coiffure ne permettent encore pas à la coiffure de résister de manière satisfaisante aux différents mouvements naturels de la vie comme la marche, les mouvements de tête ou les coups de vent.

Les polymères utilisés pour la formulation de ces produits capillaires sont des polymères filmogènes anioniques, cationiques, amphotères ou non ioniques, qui conduisent à la formation de films possédant un caractère plus ou moins dur et cassant.

Le document FR 2 750 601 décrit des compositions capillaires comprenant des agents de coloration cristaux liquides, un copolymère acide polyacrylique et un copolymère acétate de vinyle/acide crotonique.

Les documents WO 00/30593 et WO 00/30594 décrivent des compositions de coiffage comprenant, dans un milieu cosmétiquement acceptable, au moins un polymère (A) aux caractéristiques particulières et au moins un polymère (B) choisi parmi les polymères filmogènes anioniques, cationiques ou amphotères ou certains polymères filmogènes non ioniques.

Lorsque le polymère est trop cassant, le pourcentage d'allongement à la rupture mesuré sur le film est faible, c'est-à-dire en général inférieur à 2 % et la tenue de la coiffure n'est pas assurée dans le temps.

Pour remédier à ce problème, on a déjà mélangé ces polymères avec des plastifiants et obtenu des revêtements plus souples et non friables. Toutefois, ces films sont déformables et plastiques, c'est-à-dire qu'après déformation, ils ne récupèrent que très peu de leur forme initiale. Si la tenue de la coiffure est améliorée, elle n'est pas encore satisfaisante puisque la forme de la coiffure évolue dans le temps.

Il a déjà été proposé par ailleurs d'associer entre eux plusieurs de ces polymères. Dans ce cas, la forme de la coiffure peut durer plus longtemps, mais les compositions réalisées altèrent les propriétés cosmétiques des cheveux lors de leur application. Par exemple, les cheveux deviennent rêches au toucher et sont difficiles à démêler.

On recherche donc des compositions cosmétiques, notamment pour le maintien et/ou la fixation des fibres kératiniques, et notamment de la coiffure, qui procurent à la chevelure, outre une fixation durable, de bonnes propriétés cosmétiques, notamment un bon démêlage, de la douceur et un aspect agréable.

De manière surprenante et inattendue, la Demanderesse a découvert qu'il était possible de remédier aux problèmes techniques évoqués ci-dessus en utilisant certaines associations spécifiques de polymères.

L'invention a pour objet une composition cosmétique comprenant, dans un milieu cosmétiquement acceptable:

(1) au moins un polymère (A) filmogène choisi de telle sorte qu'un film obtenu par séchage d'un mélange de ce polymère (A) avec de l'éthanol ou de l'eau, à température ambiante et à un taux d'humidité relative de 50 %, présente un profil mécanique défini par au moins:

(i) un taux d'allongement à la rupture ($\varepsilon_r$) supérieur ou égal à 300 %;
(ii) une recouvrance à 300 secondes ($R_{300}$) supérieure ou égale à 45 %;
(iii) si la recouvrance à 300 secondes ($R_{300}$) est comprise entre 45 % et 60 %, alors l'allongement à la rupture est inférieur ou égal à 1300 %; le polymère (A) étant un polymère acide acrylique/acrylate d'éthyle/acrylonitrile/acrylamide;

(2) au moins un polymère (B) épaississant réticulé comprenant au moins un monomère d'acide (méth-)acrylique, la proportion du (ou des) polymère(s) (B) dans la composition étant d'au moins 0,6 % en poids par rapport au poids total de la composition.

Un autre objet de la présente invention concerne un procédé de mise en forme ou de maintien de la coiffure comprenant la mise en oeuvre de cette composition.

Encore un autre objet de la présente invention concerne l'utilisation de cette composition pour la fabrication de compositions cosmétiques capillaires, en vue d'obtenir un maintien ou une mise en forme de la coiffure.

Les polymère (A) particulièrement visé par la présente invention est celui distribué par Goodrich sous l'appellation Hystretch V29®.

Au sens de la présente invention, on entend par film obtenu par séchage à température ambiante (22 ± 2°C) et à un taux d'humidité relative de 50 % ± 5%, le film obtenu dans ces conditions à partir d'un mélange à 6 % de matière active (m.a.) de polymère A avec de l'éthanol ou de l'eau, la quantité de mélange étant adaptée pour obtenir dans une matrice en téflon, un film d'épaisseur de $500 \pm 50$ $\mu$m. Le séchage est poursuivi jusqu'à ce que le poids du film n'évolue plus, ce qui représente environ 12 jours. Les polymères A solubles ou partiellement solubles dans l'éthanol sont testés dans l'éthanol. Les autre polymères sont testés dans l'eau sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de $22 \pm 2$ °C et un taux d'humidité relative de $50 \pm 5$ %.

Les éprouvettes sont étirées à la vitesse de 20mm/mn et la distance entre les mors est de $50 \pm 1$ mm.

Pour déterminer la recouvrance instantanée ($R_i$), on procède comme suit:

- on étire l'éprouvette de 150 % ($\varepsilon_{max}$) c'est-à-dire 1,5 fois sa longueur initiale ($l_0$)
- on relâche la contrainte en imposant une vitesse de retour égale à la vitesse de traction, soit 20mm/mn et on mesure l'allongement de l'éprouvette en pourcentage , après retour à charge nulle ($\varepsilon_i$).

La recouvrance instantanée en % ($R_i$) est donnée par la formule ci-après:

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300}$).

La recouvrance en % à 300 secondes ($R_{300}$) est donnée par la formule ci-après:

$$R_{300} = ((\varepsilon_{max} - \varepsilon_{300})/ \varepsilon_{max}) \times 100$$

Les compositions selon l'invention peuvent contenir, comme polymère (B) épaississant réticulé, au moins un copolymère d'acide (méth-) acrylique réticulé, comme les Carbopol 1342 ou 1382, Pemulen TR1, Pemulen TR2 commercialisés par Goodrich.

Les compositions selon l'invention comprennent avantageusement, comme polymère (B) épaississant réticulé, au moins un homopolymère d'acide (méth-) acrylique réticulé. En particulier, on utilise, comme polymère (B), les polymères Carbopol 940, Carbopol 941, Carbopol 980, Carbopol 981, Carbopol ETD 2001, Carbopol ETD 2050, Carbopol 2984, Carbopol 5984, Carbopol Ultrez 10 commercialisés par Goodrich; Synthalen K ou Synthalen L ou Synthalen MS commercialisés par 3V; Modarez V1250 PX, Modarez V2000 PX, Viscaron A1600 PE, Viscaron A700 PE proposés par la Société Protex.

Dans les compositions conformes à l'invention, le ou les polymères (A) filmogènes sont, de préférence, présents à des concentrations comprises entre 0,05 et 20 % en poids, plus préférentiellement comprises entre 0,5 et 10 % en poids, et plus préférentiellement entre 1 et 8 % en poids par rapport au poids total de la composition.

Dans les compositions conformes à l'invention, le ou les polymères (B) épaississants réticulés sont, de préférence, présents à des concentrations comprises entre 0,6 et 2 % en poids, plus préférentiellement comprises entre 0,7 et 1,8 % en poids.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en $C_1$-$C_4$.

Parmi ces alcools, on peut citer l'éthanol, l'isopropanol. L'éthanol est particulièrement préféré.

La composition de l'invention peut également contenir au moins un additif choisi parmi les épaississants autres que ceux de l'invention, les tensioactifs, les parfums, les conservateurs, les filtres solaires, les protéines, les vitamines, les polymères non fixants, les huiles minérales, végétales ou synthétiques, les provitamines, les silicones volatiles ou non, linéaires ou cycliques, modifiées ou non et tout autre additif classiquement utilisé dans les compositions cosmétiques destinées à être appliquées sur les cheveux.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions conformes à l'invention se présentent sous la forme de compositions épaissies et notamment, sous la forme de gels.

Les compositions conformes à l'invention peuvent être appliquées sur des cheveux secs ou humides.

L'invention va être plus complètement illustrée à l'aide de l'exemple non limitatif suivant.

Tous les pourcentages sont des pourcentages relatifs en poids par rapport au poids total de la composition et m.a. signifie matière active.

| Hystretch V29 | Polymère acide acrylique/acrylate d'éthyle/Acrylonitrile/Acrylamide commercialisé par Goodrich |
| Aristoflex A | Copolymère acétate de vinyle/acide crotonique commercialisé par Hoescht |
| Synthalen K | Homopolymère d'acide acrylique réticulé commercialisé par 3 V |

EXEMPLE COMPARATIF

On réalise les compositions 1 et 2 suivantes, se présentant sous la forme de gels.

Composition 1 (invention):

| Hystretch V 29 | 3% m.a. |
| Synthalen K | 1% m.a. |
| A.M.P. | qs neutralisation du synthalen |
| conservateur | qs |
| Eau | qsp 100% |

Composition 2 (art antérieur)

| Aristoflex A | 3% m.a. |
| Synthalen K | 1% m.a. |
| A.M.P. | qs neutralisation synthalen |
| conservateur | qs |
| Eau | qsp 100% |

(a) Propriétés mécaniques des polymères filmogènes sur les cheveux

| | Hystretch V29 (invention) | Aristoflex A (art antérieur) |
|---|---|---|
| taux d'allongement à la rupture ($\varepsilon_r$) en % | 680 | 1500 |
| recouvrance instantanée ($R_i$) en %, | 80 | 28 |
| recouvrance à 300 secondes ($R_{300}$) en % | 90 | 54 |

(b) Evaluation de la tenue de la coiffure sur têtes

Sur 5 modèles, on partage la chevelure en deux côtés (droit et gauche) symétriques. Les compositions 1 et 2 sont appliquées chacune d'un côté de la tête, sur une chevelure sèche mise en forme sans application de produit au préalable. L'application des produits conduit à la formation de "méchés" c'est à dire à la formation de petites mèches de cheveux collés entre eux donnant un aspect mouillé à la chevelure.

On note juste après l'application l'effet de fixation. Les modèles reviennent 5 heures après application, et on note à ce moment-là le niveau de fixation de chaque côté de la chevelure. Cela revient à comparer la tenue des méchés 5 heures après leur formation.

Composition 1 : les méchés tiennent très bien
Composition 2 : la plupart des méchés sont défaits. La coiffure ne ressemble plus à ce qu'elle était juste après application.

On note les propriétés cosmétiques des cheveux lorsque les modèles reviennent 5 heures après application :

| Composition | Démêlage | Douceur | Toucher |
|---|---|---|---|
| 1 | très facile | Cheveux doux | correct |
| 2 | moyennement facile | Cheveux un peu rêches | chargé |

Les cheveux traités par la composition 1 présentent de meilleures propriétés cosmétiques que ceux traités par la composition 2.

(c) <u>Evaluation de la tenue de la coiffure sur perruques</u>

On peut aussi déterminer les performances de tenue des compositions 1 et 2 en suivant dans le temps l'évolution de " méchés " sous agitation mécanique.
Les supports utilisés sont des perruques d'environ 20 g de cheveux eurochatains d'une longueur de 20 cm.
On applique les compositions sur les perruques dont les cheveux sont lavés et mis en forme, à raison de 2 g de gel sur chaque perruque.
On laisse sécher 1 heure à température ambiante.
On agite ensuite les perruques pendant une heure par des mouvements rotatifs en atmosphère conditionnée à 50% d'humidité relative, 24°C.
On note la cohésion des méchés après cette agitation.
La perruque traitée par la formule 1 a des méchés beaucoup plus résistants et mieux formés après une heure d'agitation que la formule 2.
Il en résulte que les compositions conformes à l'invention procurent de meilleurs résultats en terme tenue dans le temps de la coiffure que les compositions conformes à l'art antérieur.
On note les propriétés cosmétiques des cheveux sur perruques après agitation :

| composition | Démêlage | Douceur | toucher |
|---|---|---|---|
| 1 | Très facile | Cheveux doux | correct |
| 2 | Moyennement facile | Cheveux un peu rêches | chargé |

Les cheveux traités par la composition 1 selon l'invention présentent de meilleures propriétés cosmétiques que ceux traités par la composition 2.

**Revendications**

1. Composition cosmétique comprenant, dans un milieu cosmétiquement acceptable:

   (1) au moins un polymère (A) filmogène choisi de telle sorte qu'un film obtenu par séchage d'un mélange de ce polymère (A) avec de l'éthanol ou de l'eau, à température ambiante et à un taux d'humidité relative de 50 %, présente un profil mécanique défini par au moins:

   (i) un taux d'allongement à la rupture ($\varepsilon_r$) supérieur ou égal à 300 %;
   (ii) une recouvrance à 300 secondes ($R_{300}$) supérieure ou égale à 45 %;
   (iii) si la recouvrance à 300 secondes ($R_{300}$) est comprise entre 45 % et 60 %, alors l'allongement à la rupture est inférieur ou égal à 1300 %;

(2) au moins un polymère (B) épaississant réticulé comprenant au moins un monomère d'acide (méth-)acrylique, la proportion du (ou des) polymère(s) épaississant(s) (B) dans la composition étant d'au moins 0,6 % en poids par rapport au poids total de la composition;

le polymère (A) étant un polymère acide acrylique/acrylate d'éthyle/acrylonitrile/acrylamide.

2. Composition selon la revendication 1, **caractérisée par le fait que** le polymère (B) épaississant réticulé est un homopolymère d'acide (méth-) acrylique réticulé.

3. Composition selon l'une quelconque des revendications précédentes, **caractérisée par le fait que** le ou les polymères filmogènes (A) sont présents à des concentrations comprises entre 0,05 et 20 % en poids, plus préférentiellement comprises entre 0,5 et 10 % en poids, et plus préférentiellement entre 1 et 8 % en poids par rapport au poids total de la composition.

4. Composition selon l'une quelconque des revendications précédentes, **caractérisée par le fait que** le ou les polymères (B) épaississants réticulés sont présents à des concentrations comprises entre 0,6 et 2 % en poids, plus préférentiellement comprises entre 0,7 et 1,8 % en poids.

5. Composition selon l'une quelconque des revendications précédentes, **caractérisée par le fait qu'**elle contient en outre au moins un additif choisi parmi les épaississants autres que ceux de l'invention, les tensioactifs, les parfums, les conservateurs, les filtres solaires, les protéines, les vitamines, les polymères non fixants, les huiles minérales, végétales ou synthétiques, les silicones volatiles ou non, linéaires ou cycliques, modifiées ou non et tout autre additif classiquement utilisé dans les compositions cosmétiques destinées à être appliquées sur les cheveux.

6. Dispositif aérosol contenant une composition selon l'une quelconque des revendications précédentes.

7. Procédé de maintien ou de mise en forme de la coiffure, **caractérisé par le fait qu'**il comprend la mise en oeuvre d'une composition conforme à l'une quelconque des revendications 1 à 5.

8. Utilisation d'une composition selon l'une quelconque des revendications 1 à 5, pour la fabrication d'un produit cosmétique capillaire, en vue de maintenir et/ou de fixer la coiffure.

**Claims**

1. Cosmetic composition comprising, in a cosmetically acceptable medium:

   (1) at least one film-forming polymer (A) chosen such that a film obtained by drying a mixture of this polymer (A) with ethanol or water, at room temperature and at a relative humidity of 50%, has a mechanical profile defined by at least:

   (i) a degree of elongation at break ($\varepsilon_r$) of greater than or equal to 300%;
   (ii) a recovery at 300 seconds ($R_{300}$) of greater than or equal to 45%;
   (iii) if the recovery at 300 seconds ($R_{300}$) is between 45% and 60%, then the elongation at break is less than or equal to 1300%;

   (2) at least one crosslinked thickening polymer (B) comprising at least one (meth)acrylic acid monomer, the proportion of the thickening polymer(s) (B) in the composition being at least 0.6% by weight relative to the total weight of the composition;

   the polymer (A) being an acrylic acid/ethyl acrylate/acrylonitrile/aryl amide polymer.

2. Composition according to Claim 1, **characterized in that** the crosslinked thickening polymer (B) is a crosslinked (meth)acrylic acid homopolymer.

3. Composition according to either of the preceding claims, **characterized in that** the film-forming polymer(s) (A) is (are) present at concentrations of between 0.05% and 20% by weight, more preferably between 0.5% and 10% by weight and even more preferably between 1% and 8% by weight, relative to the total weight of the composition.

4. Composition according to any one of the preceding claims, **characterized in that** the crosslinked thickening polymer (s) (B) is (are) present at concentrations of between 0.6% and 2% by weight and more preferably between 0.7% and 1.8% by weight.

5. Composition according to any one of the preceding claims, **characterized in that** it also contains at least one additive chosen from thickeners other than those of the invention, surfactants, fragrances, preserving agents, sunscreens, proteins, vitamins, non-fixing polymers, mineral, plant or synthetic oils, volatile or non-volatile, linear or cyclic, modified or unmodified silicones and any other additive conventionally used in cosmetic compositions intended to be applied to the hair.

6. Aerosol device containing a composition according to any one of the preceding claims.

7. Process for holding or shaping the hairstyle, **characterized in that** it comprises the use of a composition in accordance with any one of Claims 1 to 5.

8. Use of a composition according to any one of Claims 1 to 5 for the manufacture of a cosmetic haircare product, in order to hold and/or fix the hairstyle.

**Patentansprüche**

1. Kosmetische Zusammensetzung, die in einem kosmetisch akzeptablen Medium enthält:

   (1) mindestens ein filmbildendes Polymer (A), das so ausgewählt wird, dass ein Film, der durch Trocknen eines Gemischs dieses Polymers (A) mit Ethanol oder Wasser bei Umgebungstemperatur und einer relativen Feuchtigkeit von 50 % erhalten wird, ein mechanisches Profil aufweist, das mindestens definiert ist durch

   (i) eine Bruchdehnung ($\varepsilon_B$) von 300 % oder darüber;
   (ii) eine Rückstellung nach 300 s ($R_{300}$) von 45 % oder darüber;
   (iii) wenn die Rückstellung nach 300 s ($R_{300}$) im Bereich von 45 bis 60 % liegt, ist die Bruchdehnung kleiner als oder gleich 1300 %;

   (2) mindestens ein vernetztes verdickendes Polymer (B), das mindestens ein (Meth)acrylsäure-Monomer enthält, wobei der Anteil des Polymers (B) oder der Polymere (B) in der Zusammensetzung mindestens 0,6 Gew.-%, bezogen auf das Gesamtgewicht der Zusammensetzung, beträgt,

   wobei das Polymer (A) ein Acrylsäure/Ethylacrylat/Acrylnitril/-Acrylamid-Polymer ist.

2. Zusammensetzung nach Anspruch 1, **dadurch gekennzeichnet, dass** das vernetzte verdickende Polymer (B) ein vernetztes Homopolymer von (Meth)acrylsäure ist.

3. Zusammensetzung nach einem der vorhergehenden Ansprüche, **dadurch gekennzeichnet, dass** das oder die filmbildenden Polymere (A) in Konzentrationen enthalten sind, die im Bereich von 0,05 bis 20 Gew.-%, bevorzugter im Bereich von 0,5 bis 10 Gew.-% und noch bevorzugter im Bereich von 1 bis 8 Gew.-% liegen, bezogen auf das Gesamtgewicht der Zusammensetzung.

4. Zusammensetzung nach einem der vorhergehenden Ansprüche, **dadurch gekennzeichnet, dass** das oder die vernetzten verdickenden Polymere (B) in Konzentrationen enthalten sind, die im Bereich von 0,6 bis 2 Gew.-%, bevorzugter im Bereich von 0,7 bis 1,8 Gew.-%, liegen.

5. Zusammensetzung nach einem der vorhergehenden Ansprüche, **dadurch gekennzeichnet, dass** sie außerdem mindestens einen Zusatzstoff enthält, der unter den Verdickungsmitteln, die von den erfindungsgemäßen Verdickungsmitteln verschieden sind, den grenzflächenaktiven Stoffen, den Parfüms, den Konservierungsmitteln, den Sonnenschutzfiltern, den Proteinen, den Vitaminen, den nicht festigend wirkenden Polymeren, den Mineralölen, den pflanzlichen oder synthetischen Ölen, den flüchtigen oder nicht-flüchtigen, geradkettigen oder zyklischen, modifizierten oder nicht modifizierten Siliconen und allen sonstigen Zusatzstoffen, die herkömmlicherweise in kosmetischen Zusammensetzungen verwendet werden, die für die Anwendung auf den Haaren vorgesehen sind, ausgewählt wird.

6. Aerosolvorrichtung, die eine Zusammensetzung nach einem der vorhergehenden Ansprüche enthält.

7. Verfahren zur Erhaltung oder Gestaltung der Frisur, **dadurch gekennzeichnet, dass** es die Anwendung einer Zusammensetzung nach einem der Ansprüche 1 bis 5 umfasst.

8. Verwendung einer Zusammensetzung nach einem der Ansprüche 1 bis 5 für die Herstellung eines kosmetischen Haarbehandlungsprodukts, um die Frisur zu erhalten und/ oder zu festigen.